Europäisches Patentamt

European Patent Office

Office européen des brevets

(19)

(11) Publication number: 0 391 400

A2

(12) # EUROPEAN PATENT APPLICATION

(21) Application number: 90106492.3

(51) Int. Cl.⁵: C12N 9/96, C12N 9/72

(22) Date of filing: 05.04.90

(30) Priority: 07.04.89 JP 86932/89

(43) Date of publication of application:
10.10.90 Bulletin 90/41

(84) Designated Contracting States:
BE CH DE DK ES FR GB IT LI NL SE

(71) Applicant: THE GREEN CROSS CORPORATION
3-3, Imabashi 1-chome Chuo-ku
Osaka-shi Osaka 541(JP)

(72) Inventor: Morimoto, Kazuo, c/o The Green
Cross Corporation
Osadano Plant, 2-11, Osadano-cho
Fukuchiyama-shi, Kyoto 620(JP)
Inventor: Narita, Shusaku, c/o The Green
Cross Corporation
Osadano Plant, 2-11, Osadano-cho
Fukuchiyama-shi, Kyoto 620(JP)
Inventor: Nishikawa, Masaru, c/o The Green
Cross Corporation
Osadano Plant, 2-11, Osadano-cho
Fukuchiyama-shi, Kyoto 620(JP)
Inventor: Takechi, Kazuo, c/o The Green
Cross Corporation
Central Research Lab., 2-1180-1,
Shodaiohtani
Hirakata-shi, Osaka 573(JP)

(74) Representative: von Kreisler, Alek,
Dipl.-Chem. et al
Patentanwälte Von Kreisler-Selting-Werner
Deichmannhaus am Hauptbahnhof
D-5000 Köln 1(DE)

(54) Urokinase precursor-stabilizing method and dried composition.

(57) A method for stabilizing urokinase precursors which comprises adding at least one species of stabilizer selected from the polar amino acids and salts thereof to an urokinase precursor and a dried urokinase precursor composition which comprises an urokinase precursor as the main ingredient and at least one species selected from the polar amino acids and salts thereof as the stabilizing agent are disclosed. According to the present invention, the urokinase precursors can be stabilized even under the conditions in which the urokinase precursors may be inactivated, such as purification, formulation, lyophilization and storage periods for the urokinase precursors.

## Urokinase Precursor-Stabilizing Method and Dried Composition

### FIELD OF THE INVENTION

This invention relates to a stable dried composition of an urokinase precursor and a method for stabilizing urokinase precursors.

### BACKGROUND OF THE INVENTION

Although urokinase precursors secreted from human renal cells by themselves do not exhibit urokinase activities, they exhibit remarkable urokinase activities when they are subjected to action of a proteinase such as plasmin.

Urokinase precursors, which have a strong affinity for fibrins, can reach fibrins of thrombus without acting on fibrinogens (decomposing fibrins) in blood plasma and exhibit urokinase activities by a slight amount of plasmin in the thrombus. (See European Patent Application Publication No. 139447.)

Thus, since urokinase precursors have characteristics in that they cause fibrinolysis only at the thrombus site, whereby selective and efficient lysis of thrombus is caused, urokinase precursors have been expected to be used as a novel therapeutic agent for vascular embolus diseases.

However, though urokinase precursors are relatively stable in the crude state and when maintained in a neutral solvent at a high concentration in the purification process, they are unstable in a highly purified state or in a dried state, especially in a freeze-dried state.

### SUMMARY OF THE INVENTION

An object of the invention is to provide a stable dried urokinase precursor composition.

Another object of this invention is to provide a method for stabilizing urokinase precursors.

The present inventors conducted intensive studies to solve the above-mentioned problems, and as a result, found that urokinase precursors, specifically dried urokinase precursors could be stabilized by addition of at least one species selected from polar amino acids and salts thereof to the urokinase precursors, which resulted in the completion of this invention.

That is, this invention relates to the following 1) and 2).

1) A method for stabilizing urokinase precursors which comprises adding at least one species of stabilizer selected from polar amino acids and salts thereof to an urokinase precursor.

2) A dried urokinase precursor composition which comprises an urokinase precursor as the main ingredient and at least one species selected from polar amino acids and salts thereof as the stabilizing agent.

### DETAILED DESCRIPTION OF THE INVENTION

(Urokinase precursors)

Though urokinase precursors used in the present inven tion have scarecely possess any fibrinolysis activities, when they are converted into urokinase by treatment with an enzyme such as plasmin, they display fibrinolysis activities. Also, even in the presence of fibrin, urokinase precursors exhibit some fibrinolysis activities.

Representative examples of the urokinase precursors to be used in the present invention include, among others, single-chained peptides having a molecular weight of 50,000 - 55,000.

As such single-chained urokinase precursors, mention can be made of, for example, a peptide containing 411 constituent amino acids (European Patent Application Publication No. 139447).

The origins of the aforementioned urokinase precursors are not limited, and the urokinase precursors can be obtained, for example, in accordance with the cell culture methods which are described in European Patent Application Publication No. 139447, etc., the genetic engineering methods which are described in European Patent Application Publication Nos. 154272, 232544, 265874, etc. and other methods.

The urokinase precursors in the present invention are not limited to the above-mentioned ones but generically encompass their derivatives. As such derivatives, mention can be made of, for example, protein molecules which are defective in the entire region of or a portion of the epidermal growth factor domain of urokinase precursors, or said entire region or a portion is substituted by other amino acid residues; etc. Accordingly, unless otherwise specified, uroki nase precursors mean urokinase precursors per se and also the aforementioned urokinase precursor derivatives throughout the present specification.

These urokinase precursor derivatives usually have a molecular weight of about 40,000 - 50,000 and a single-chained peptide structure as the urokinase precursors per se described above. Moreover, urokinase precursor derivatives exhibit fibrinolysis activities in the same manner as the above-mentioned urokinase precursors per se.

The derivatives can be prepared, for example, by a genetic engineering procedure (European Patent Application Publication No. 253541, etc.).

The effects of this invention are especially remarkable for urokinase precursors which are purified to a high extent. For example, mention can be made of urokinase precursors having a specific activity of about 80,000 - 200,000 IU/mg protein ["IU" is an abbreviation of international unit of urokinase (UK). By "1 IU/ml" corresponding to the UK activity exhibited by 1 ml of a precursor which has been treated with plasmin, it is meant that 1 ml of a solution of a precursor exhibit the same activity as UK 1 IU when treated with plasmin. The same shall apply hereinafter.]

(Stabilizer)

As the polar amino acids to be used in the present invention, mention may be made of, for example, glutamic acid, aspartic acid, arginine, lysine, histidine, etc.

As the salts of polar amino acids, preferred are sodium glutamate and alkali metal aspartates (e.g. sodium salt).

(Amount of stabilizer to be added)

A polar amino acid or a salt thereof as a stabilizer can be added in a proportion of 5 - 30 mg relative to 10,000 -250,000 IU of the urokinase precursor used.

Also, dextran may be added as another stabilizer. As such dextrans, there are mentioned, for example, those of a molecular weight of 1,000 - 100,000. The dextran is added in a proportion of 10 - 50 mg relative to 10,000 - 250,000 IU of the urokinase precursor used.

In accordance with the present invention, the abovementioned stabilizers can be added at anytime when urokinase precursors may be inactivated, such as purification, formulation, lyophilization and storage periods for urokinase precursors, and exhibit their stabilizing effects on them.

The present invention is more definitely and specifically described by the following experiment examples and working examples, but they are not construed to limit the scope of the invention.

The activities of the urokinase precursors was measured with a synthetic substrate (Glt-Gly-Arg-MCA) after activated with plasmin.

Experiment Example 1

Prepared was 25,000 IU/ml of a solution of purified urokinase precursor (specific activity of 145,000 IU/mg protein) in a 0.10 M phosphate buffer solution as a solvent. To the obtained solution were added various additives (including a polar amino acid or a salt thereof) in the total amount of 6.4 mg/ml, and the mixture was lyophilized. As the control, used were the lyophilized preparations of a solution of a purified urokinase precursor without any additives and a solution with human albumin alone added.

The titers (%) of the residual urokinase precursors immediately after lyophilization and after storage at 50°C for 3 months were measured, and the results are shown in Table 1.

3

Table 1

| Added amino acid (6.4 mg each) | | Residual activity immediately after lyophilization (%) | Residual activity after storage at 50°C for 3 months (%) |
|---|---|---|---|
| Present Invention | Aspartic acid | 96 | 60 |
| | Glutamic acid | 96 | 60 |
| | Sodium glutamate | 96 | 61 |
| | Arginine | 96 | 63 |
| | Lysine | 96 | 60 |
| | Histidine | 96 | 56 |
| Control | – | 94 | 2 |

Experiment Example 2

In the same manner as in Experiment Example 1, the lyophilized preparations were obtained by mixing arginine and sodium glutamate in various proportions.

The titers (%) of the lyophilized preparations immediately after lyophilization and after storage at 50°C for 3 months were measured. The results are as shown in Table 2.

Table 2

| Amino acid | | Residual activity immediately after lyophilization (%) | Residual activity after storage at 50°C for 3 months (%) |
|---|---|---|---|
| Arginine | not added | 94 | 2 |
| | 3.2 mg/ml | 95 | 54 |
| | 6.4 mg/ml | 96 | 63 |
| Sodium glutamate | not added | 94 | 2 |
| | 3.2 mg/ml | 95 | 53 |
| | 6.4 mg/ml | 96 | 61 |

The titers shown in Tables 1 and 2 are the residual activities based on the titers before the

4

aforementioned treatment taken as 100.

Example 1

In 1 ml of a 0.1 M phosphate buffer (pH 7.0) were dissolved 25,000 IU of purified urokinase precursors (the specific activity of 145,000 IU/mg protein) and 6.4 mg of arginine, and the solution was subjected to sterile filtration. Thereafter, a vial was filled with the solution, which was lyophilized to obtain an urokinase precursor preparation.

Example 2

By conducting the same procedure as in Example 1, there was obtained an urokinase precursor preparation containing 6.4 mg of sodium glutamate.

Example 3

By conducting the same procedure as in Example 1 with the use of 50,000 IU of a purified urokinase precursor (the specific activity of 135,000 IU/mg protein) and 12.8 mg of arginine, an urokinase precursor preparation was obtained.

<Reference Example : Production Example>

Cultured human renal cells were cultivated for 3 days in a culture medium free of serum with 0.1% human serum albumin added, and the culture broth was centrifuged and the supernatant was freezed and stored. After the pooled culture supernatant was adjusted to pH 5.5, it was subjected to CM-Sephadex C-50. The column was washed with a 0.16 M phosphate buffer (pH 5.5), and thereafter the adsorbed urokinase precursor of the present invention was eluted with a 0.16 M phosphate buffer (pH 8.5).

In the meantime, clones which had a high productivity of antibody against the present urokinase precursor or the urinary urokinase were selected from among hybridomas obtained by fusing, with the use of polyethyleneglycol, mouse BALB/c spleen cells and mouse myeloma cells which had been in advance immunized by the present urokinase precursor or the urinary urokinase. From the culture broth of the fused cells, anti-urokinase monoclonal antibodies were recovered. Thus obtained monoclonal antibodies were fixed on BrCN-activated Sepharose 4B (Pharmacia Corp.)

This monoclonal antibody column was equilibrated with a 0.1 M phosphate buffer containing 0.4 M NaCl (pH 7.0), with which the above-mentioned urokinase precursor-containing eluate was brought into contact. After the column was washed with a 0.1 M phosphate buffer containing 0.4 M NaCl (pH 7.0), the adsorbed urokinase precursor was eluted with a 0.2 M aqueous solution of glycine-HCl containing 0.5 M NaCl (pH 2.5). The eluate was neutralized and then passed through the carrier on which anti-mouse IgG rabbit IgG had been immobilized, to remove a leaked-out slight amount of mouse IgG. After the passed solution was subjected to filtration for sterilization, it was lyophilized to obtain the objective highly purified urokinase precursor having a specific activity of not less than 145,000 IU/mg.

This purified product showed a band of the molecular weight of 50,000 - 55,000 in the SDS-polyacrylamide gel electrophoresis.

**Claims**

1. A method for stabilizing urokinase precursors which comprises adding at least one species of stabilizer selected from polar amino acids and salts thereof to an urokinase precursor.

2. A method for stabilizing urokinase precursors as claimed in Claim 1, characterized in that the urokinase precursor has a molecular weight of 50,000 - 55,000 and a single-chained peptide structure.

3. A method for stabilizing urokinase precursors as claimed in Claim 2 wherein the number of the constituent amino acids of the urokinase precursor is 411.

4. A method for stabilizing urokinase precursors as claimed in Claim 1 wherein the urokinase precursor is selected from among those which are defective in the entire region of or a portion of the epidermal growth factor domain and those whose entire region of or a portion of the epidermal growth factor domain is substituted by other amino acid residues.

5. A method for stabilizing urokinase precursors as claimed in Claim 1 wherein the urokinase precursor is highly purified.

6. A method for stabilizing urokinase precursors as claimed in Claim 1 wherein the urokinase precursor has a specific activity of about 80,000 - 200,000 IU/mg protein.

7. A method for stabilizing urokinase precursors as claimed in Claim 1 wherein the polar amino acid is selected from among glutamic acid, aspartic acid, arginine, lysine and histidine.

8. A method for stabilizing urokinase precursors as claimed in Claim 1 wherein a salt of the polar amino acid is selected from among alkali metal glutamates and alkali metal aspartates.

9. A method for stabilizing urokinase precursors as claimed in Claim 1 wherein the stabilizer selected from among the polar amino acids or salts thereof is added in a proportion of 5 - 30 mg relative to 10,000 - 250,000 IU of the urokinase precursor.

10. A method for stabilizing urokinase precursors as claimed in Claim 1 wherein dextran is used as a co-stabilizer.

11. A dried urokinase precursor composition which comprises an urokinase precursor as the main ingredient and at least one species selected from the polar amino acids and salts thereof as the stabilizing agent.

12. A dried urokinase precursor composition as claimed in Claim 11, characterized in that the urokinase precursor has a molecular weight of 50,000 - 55,000 and a single-chained peptide structure.

13. A dried urokinase precursor composition as claimed in Claim 12 wherein the number of the constituent amino acids of the urokinase precursor is 411.

14. A dried urokinase precursor composition as claimed in Claim 11 wherein the urokinase precursor is selected from among those which are defective in the entire region of or a portion of the epidermal growth factor domain and those whose entire region of or a portion of the epidermal growth factor domain is substituted by other amino acid residues.

15. A dried urokinase precursor composition as claimed in Claim 11 wherein the urokinase precursor is highly purified.

16. A dried urokinase precursor composition as claimed in Claim 11 wherein the urokinase precursor has a specific activity of about 80,000 - 200,000 IU/mg protein.

17. A dried urokinase precursor composition as claimed in Claim 11 wherein the polar amino acid is selected from among glutamic acid, aspartic acid, arginine, lysine and histidine.

18. A dried urokinase precursor composition as claimed in Claim 11 wherein the salt of the polar amino acid is selected from among alkali metal glutamates and alkali metal aspartates.

19. A dried urokinase precursor composition as claimed in Claim 11 wherein the stabilizer selected from among the polar amino acids or salts thereof is added in a proportion of 5 - 30 mg relative to 10,000 - 250,000 IU of the urokinase precursor.

20. A dried urokinase precursor composition as claimed in Claim 11 wherein dextran is used as a co-stabilizer.